# EUROPÄISCHE PATENTSCHRIFT

(11) **EP 2 845 609 B1**
(45) Veröffentlichungstag und Bekanntmachung des Hinweises auf die Patenterteilung: **08.06.2016**
(21) Anmeldenummer: 14183893.8
(22) Anmeldetag: 08.09.2014
(51) Int. Cl.: A61L 2/08, B29C 71/04, B29C 49/42, B65B 55/08, H01J 29/06

(54) **Vorrichtung und Verfahren zum Sterilisieren von Behältnissen mit Wartungsmöglichkeit**
Apparatus and method for sterilising containers with possibility of maintenance
Dispositif et procédé de stérilisation de récipients avec une possibilité d'entretien

(30) Priorität: 06.09.2013 DE 102013109794
(43) Veröffentlichungstag der Anmeldung: 11.03.2015
(73) Patentinhaber: Krones AG, 93073 Neutraubling (DE)
(72) Erfinder: Scheuren, Hans, 93073 Neutraubling (DE); Knott, Josef, 93073 Neutraubling (DE); Seidenberg, Katharina, 93073 Neutraubling (DE); Neubauer, Michael, 93073 Neutraubling (DE)
(74) Vertreter: Bittner, Bernhard

(56) Entgegenhaltungen:
- WO-A1-2013/058205
- WO-A1-2013/092735
- WO-A2-2009/095182
- DE-A1-102011 055 553

## Beschreibung

Die vorliegende Erfindung bezieht sich auf eine Vorrichtung und ein Verfahren zum Sterilisieren von Behältnissen. Im Bereich der Getränke herstellenden Industrie ist es oftmals üblich, abzufüllende Behältnisse, insbesondere vor ihrer Befüllung, zu sterilisieren. Während in der Vergangenheit zu dieser Sterilisierung üblicherweise Substanzen wie Wasserstoffperoxid oder Peressigsäure eingesetzt wurden, geht man in jüngerer Zeit auch dazu über, diese Sterilisation der Behältnisse durch Bestrahlung und insbesondere durch Bestrahlung mit Elektronen oder Ladungsträgern durchzuführen. Diese Bestrahlung durch Ladungsträger ist dabei sehr effizient, hat jedoch als unerwünschten Nebeneffekt die Entstehung unerwünschter Strahlung und insbesondere von Röntgenstrahlen zur Folge. Um die Sicherheit für Anlagenteile und auch Benutzer zu gewährleisten ist es daher aus dem Stand der Technik bekannt, dass die Transportpfade, entlang derer die Kunststoffbehältnisse gefördert werden, abgeschirmt werden, um so das Entweichen von Röntgenstrahlung zu vermeiden. Bei den Kunststoffbehältnissen kann es sich insbesondere um Vorformlinge/Preformen und/oder Flaschen handeln.

DE102011055553 offenbart eine Elektronenstrahl-Behälterdesinfektionsvorrichtung mit abhebbarem Deckelteil zur Vereinfachung der Zugänglichkeit für Wartungszwecke.

Damit besteht oftmals eine sicherheitstechnische Herausforderung bei der Konstruktion, dem Bau und dem Betrieb eines derartigen Sterilisators darin, diesen hermetisch und strahlungssicher zu gestalten bzw. insbesondere dessen mit Strahlung beaufschlagten Bereich abzusichern.

Dieser Sicherheitsbereich bei derartigen Vorrichtungen, insbesondere im Verpackungs- und Lebensmittelbereich muss daher zwei an sich entgegengesetzten Anforderungen genügen. Zum einen muss die Abschirmfunktion sichergestellt werden, zum anderen sollte jedoch auch, insbesondere für Wartungszwecke eine möglichst günstige Zugänglichkeit in diesem Bereich gegeben sein. Im internen Stand der Technik der Anmelderin sind daher teilweise mehrere Revisionsöffnungen vorgesehen, welche eine Zugänglichkeit von Benutzern ermöglichen. Je mehr derartige Wartungsöffnungen jedoch vorgesehen werden, desto umfangreicher werden die notwendigen Sicherheits- und Kontrollmaßnahmen zur Überwachung dieser Anbindungen. Je weniger Öffnungen andererseits vorgesehen werden, desto schlechter ist der Zugang zu dem Sicherheitsbereich.

Der vorliegenden Erfindung liegt daher die Aufgabe zugrunde, einerseits einen günstigen Abschirmeffekt mit andererseits einer günstigen Zugänglichkeit für Wartungszwecke zu ermöglichen. Anders ausgedrückt, liegt der Erfindung die Aufgabe zugrunde, möglichst eine Verbesserung zwischen der Zugänglichkeit und der Sicherheit für eine derartige Verpackungsentkeimung mittels Ladungsträger und insbesondere mittels Elektronen zu verwirklichen. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältnissen ist im Anspruch 1 definiert.

Daneben weist die Vorrichtung eine Abschirmeinrichtung auf, welche zum Abschirmen von entstehender Strahlung und insbesondere von während des Sterilisationsprozesses und/oder während der Beaufschlagung der Kunststoffbehältnisse mit Elektronenstrahlung entstehender Strahlung auftritt, dient. Weiterhin umgibt die Abschirmeinrichtung wenigstens abschnittsweise den Transportpfad der Behältnisse. Dabei ist es möglich, dass sich diese Abschirmeinrichtung wenigstens abschnittsweise entlang des Transportpfades und/oder seitlich neben dem Transportpfad der Behältnisse der Behältnisse erstreckt. Unter einer Erstreckung entlang des Transportpfades muss dabei jedoch nicht zwangsläufig verstanden werden, dass beispielsweise die Abschirmeinrichtung in gleicher Weise gekrümmt ist wie der Transportpfad. So kann es beispielsweise ausreichend sein, wenn sich eine Wandung mit gegebenenfalls auch variierenden Abschnitten entlang des Transportpfades erstreckt. So wäre es beispielsweise auch möglich, dass eine Wandung, die sich entlang eines geradlinigen Abschnitts erstreckt, zur Abschirmung einer gekrümmten Transportbahn der Kunststoffvorformlinge dient. Unter einer seitlichen Anordnung wird verstanden, dass ausgehend von dem Transportpfad in einer horizontalen Ebene wenigstens abschnittsweise diese Wandung vorgesehen ist.

Vorteilhaft handelt es sich bei der Abschirmeinrichtung wenigstens abschnittsweise um eine Wandung und insbesondere eine Wandung, welche aus einem Material hergestellt ist, welches Röntgenstrahlung abschirmt, wie beispielsweise Blei in ausreichender Dicke. Vorteilhaft schirmt diese Wandung den Transportpfad der Kunststoffvorformlinge in wenigstens einer zu dem Transportpfad senkrecht stehenden Richtung ab. Vorteilhaft ist jedoch ein Verlauf dieser Wandung wenigstens abschnittsweise an einen Verlauf des Transportpfads angepasst.

Daneben kann jedoch die Wandung auch so angeordnet sein, dass sie auch diejenige Röntgenstrahlung abschirmt, welche direkt von den einzelnen Sterilisationseinrichtungen ausgeht. Dabei ist es möglich und bevorzugt, dass die Abschirmeinrichtung den Transportpfad der Behältnisse um Wesentlichen vollständig (mit Ausnahme etwa von Zuführ- und Abführöffnungen) umgibt.

Erfindungsgemäß weist die Abschirmeinrichtung eine erste Wandung auf, welche seitlich neben dem Transportpfad der Behältnisse angeordnet ist sowie eine zweite Wandung, welche unterhalb des Transportpfades der Behältnisse und damit insbesondere auch unterhalb der transportieren Behältnisse angeordnet ist, wobei wenigstens die zweite Wandung gegenüber dem (geometrischen) Transportpfad der Behältnisse bewegbar ist. Vorteilhaft liegt dabei die besagte zweite Wandung in einer vertikalen Richtung unterhalb des Transportpfades der Kunststoffbehältnisse. Der Transportpfad der Behältnisse ist dabei vorteilhaft stationär.

Vorteilhaft wird durch die besagten Wandungen ein Isolator gebildet, innerhalb dessen die Kunststoffbehältnisse transportiert werden. Es wird damit vorteilhaft im Rahmen der Erfindung vorgeschlagen, diesen Isolator so auszugestalten, dass dieser in voller Gesamtheit oder wenigstens zu Teilen geöffnet werden kann und insbesondere nach unten geöffnet werden kann und bevorzugt durch eine Verfahrbewegung eines Bodenbereichs nach unten geöffnet werden kann. In diesem Falle ist daher bevorzugt lediglich eine von sechs möglichen Ebenen im Gegensatz zu einem bevorzugt feststehenden Rest abzutrennen. Auf diese Weise kann die Zugänglichkeit für Wartungen und dergleichen verbessert werden und gleichfalls kann ein günstiger Abschirmeffekt in einem geschlossenen Zustand dieses Isolators erreicht werden. Damit handelt es sich bei der zweiten Wandung bevorzugt um eine Bodenwandung.

Bevorzugt weist die Vorrichtung damit einen Isolator auf, innerhalb dessen die Kunststoffbehältnisse transportiert werden. Dieser Isolator kann, insbesondere zu Wartungszwecken, geöffnet werden. Insbesondere kann dieser Isolator in genau einer Ebene bzw. zu einer Richtung hin geöffnet werden. Bevorzugt weist die Abschirmeinrichtung bzw. die Strahlungsabschirmeinrichtung einen während des Transports der Behältnisse entlang des Transportpfades stationären Anteil auf. Bevorzugt weist die Strahlungsabschirmungseinrichtung auch einen während des Transports der Behältnisse entlang des Transportpfades beweglichen Teil und insbesondere gegenüber dem stationären Teil beweglichen Teil auf.

Bei einer weiteren vorteilhaften Ausführungsform weist die Vorrichtung eine Innenbeaufschlagungseinrichtung zum Sterilisieren zumindest eines Abschnitts einer Innenwandung der Behältnisse auf. Vorteilhaft weist die Vorrichtung auch eine Außenbeaufschlagungseinrichtung zum Sterilisieren zumindest eines Abschnitts einer Außenwandung der Behältnisse auf. Vorteilhaft weist eine Vorrichtung zum Sterilisieren der Innenwandung der Behältnisse wenigstens zeitweise eine geringere Beschleunigungsspannung auf, als eine Außenbeaufschlagungseinrichtung zum Sterilisieren einer Außenwandung der Kunststoffbehältnisse.

Bevorzugt ist die Vorrichtung zur Außensterilisierung der Kunststoffvorformlinge gegenüber der Bewegung der Kunststoffvorformlinge stationär angeordnet. Bei einer weiteren vorteilhaften Ausführungsform weist die Innenbehandlungseinrichtung wenigstens einen stangenartigen Körper auf, der in einen Innenraum der Behältnisse durch deren Mündung hindurch einführbar ist. Weiterhin weist bevorzugt diese Innenbehandlungseinrichtung eine Beschleunigungseinrichtung zum Beschleunigen von Ladungsträgern auf, sowie auch ein Austrittsfenster, welches derart beschaffen ist, dass es durch eine Mündung der Kunststoffbehältnisse hindurch in diese einführbar ist. Bevorzugt handelt es sich bei den Ladungsträgern um geladene Teilchen und insbesondere um Elektronen.

Vorteilhaft weist die Vorrichtung eine Vielzahl von derartigen Innenbehandlungseinrichtungen auf. Diese sind vorteilhaft an einen beweglichen Träger angeordnet. Besonders bevorzugt handelt es sich bei dem beweglichen Träger um einen drehbaren Träger.

Vorteilhaft weist die Vorrichtung auch eine Vielzahl von Halteelementen zum Halten der Kunststoffbehältnisse auf. Bei einer weiteren vorteilhaften Ausführungsform weist wenigstens eine Innenbeaufschlagungseinrichtung ein Austrittsfenster auf, durch welches hindurch Ladungsträger und insbesondere Elektronen aus einer Leitungskammer zum Leiten dieser Ladungsträger austreten können. Vorteilhaft handelt es sich bei diesem Austrittsfenster um eine Folie und insbesondere um eine Titanfolie. Diese Folie weist dabei bevorzugt eine Dicke auf, welche zwischen 6 µm und 20 µm liegt, bevorzugt zwischen 8 µm und 16 µm und besonders bevorzugt zwischen 8 µm und 12 µm.

Weiterhin ist es möglich, dass die zweite Wandung einteilig ausgebildet ist, es wäre jedoch auch möglich, dass diese zweite Wandung mehrteilig ausgebildet ist, wobei besonders bevorzugt die beiden Teile auch gegenüber einander bewegt werden können. Bei einer weiteren vorteilhaften Ausführungsform weist die Abschirmeinrichtung auch eine Deckeleinrichtung auf, welche bevorzugt oberhalb des Transportpfades der Kunststoffbehältnisse und insbesondere oberhalb der Kunststoffbehältnisse angeordnet ist und auf diese Weise eine Abschirmung nach oben hin erreicht. Die Begriffe wie "unten" oder "oben" werden dabei unter Bezugnahme auf eine vertikale Richtung verstanden. Das bedeutet, dass "unten" gleichbedeutend ist mit näher am Erdmittelpunkt und "oben" gleichbedeutend ist mit weit entfernt vom Erdmittelpunkt. Bevorzugt ist diese Deckeleinrichtung in der Transportrichtung der Behältnisse beweglich angeordnet. Dabei können an dieser Deckeleinrichtung auch Halteelemente zum Halten der Kunststoffbehältnisse angeordnet sein.

Bei einer weiteren vorteilhaften Ausführungsform weist die Vorrichtung auch Dreheinrichtungen auf, welche die Kunststoffbehältnisse wenigstens abschnittsweise während deren Transport auch um eine Längsrichtung der Kunststoffbehältnisse drehen. Diese Drehung findet dabei bevorzugt zumindest in einem Abschnitt des Transportpfades statt, in dem die Kunststoffbehältnisse an ihren Außenoberflächen sterilisiert werden. Auf diese Weise kann die Außenoberfläche der Behältnisse gleichmäßiger sterilisiert werden.

Vorteilhaft ist durch eine Bewegung der zweiten Wandung wenigstens eine Sterilisationseinrichtung für Benutzereingriffe zugänglich machbar. Dies bedeutet, dass durch die besagte Bewegung der zweiten Wandung insbesondere eine Revisions- bzw. Wartungsöffnung entsteht. Insbesondere entsteht dabei eine Öffnung, die so groß ist, dass ein Benutzer durch diese Öffnung hindurch Wartungsarbeiten durchführen kann. Dabei wäre es sogar möglich, dass die Öffnung in einer so großen Größe ausgebildet wird, dass ein Benutzer durch diese Öffnung hindurch in die Vorrichtung einsteigen kann. Bevorzugt sind durch die Bewegung der zweiten Wandung mehrere Sterilisationseinrichtung zugänglich machbar.

Bevorzugt sind durch die Bewegung der zweiten Wandung sämtliche Sterilisationseinrichtungen zugänglich machbar, welche der Innensterilisation der Behältnisse dienen. Bevorzugt sind durch die Bewegung der zweiten Wandung auch sämtliche Sterilisationseinrichtungen zugänglich machbar, welche der Außensterilisation der Behältnisse dienen. Bevorzugt kann daher durch die Bewegung lediglich einer Wandung oder zweier Wandungen die vollständige Anlage für Benutzereingriffe und/oder Wartungsarbeiten zugänglich gemacht werden.

Vorteilhaft sind innerhalb der Abschirmeinrichtung auch Beaufschlagungselemente vorgesehen, mittels denen eine Sterilisation von Anlagenteilen wie etwa von Greifklammern, Transportsternen und dergleichen vorgenommen werden kann.

Bei einer weiteren vorteilhaften Ausführungsform ist die Wandung wenigstens abschnittsweise als Stützfläche für einen Benutzer ausgebildet. Dies bedeutet, dass die Wandung insbesondere geeignet ist, um das Gewicht eines Benutzers zu tragen und beispielsweise geeignet ist, Massen bzw. Gewichte bis zu wenigstens 150 kg aufzunehmen.

Dabei ist es denkbar, dass eine Antriebseinrichtung vorgesehen ist, mittels derer die Bewegung der zweiten Wandung gegenüber dem Transportpfad und insbesondere auch gegenüber der ersten Wandung realisiert werden kann.

Wie oben erwähnt, weist bevorzugt die Transporteinrichtung einen bewegbaren Träger auf, an den eine Vielzahl von Halteelementen zum Halten der Behältnisse angeordnet ist.

Bei den Halteelementen kann es sich insbesondere, aber nicht ausschließlich, um Greifklammern handeln, welche die Behältnisse im vorgegebenen Abschnitt, beispielsweise unterhalb deren Mündung bzw. unterhalb deren Tragring greifen können. Vorteilhaft ist die besagte zweite Wandung, insbesondere gegenüber diesem drehbaren Träger bewegbar und insbesondere verschiebbar. Hierbei wird unter der Bewegbarkeit verstanden, dass die Wandung insbesondere auch in einem ruhenden Zustand dieses Trägers gegenüber diesem Träger bewegbar ist, das heißt insbesondere, wenn sich der besagte insbesondere drehbare Träger nicht dreht.

Bei einer weiteren vorteilhaften Ausführungsform ist die zweite Wandung insbesondere in einer Längsrichtung der zu sterilisierenden Behältnisse bewegbar. Hierbei handelt es sich bevorzugt auch um eine Richtung, welche zu dem Transportpfad der Kunststoffbehältnisse senkrecht steht. Insbesondere ist dieser Transportpfad kreisförmig oder kreissegmentförmig angeordnet und die Richtung, in welcher die Wandung bewegbar ist, steht senkrecht zu einer durch diese Kreisbahn gebildeten Ebene. Daneben ist es jedoch auch möglich, dass die besagte zweite Wandung in einer hierzu senkrechten Richtung bewegbar ist, das heißt in einer Richtung, welche zu der Längsrichtung der zu sterilisierenden Behältnisse senkrecht steht.

Vorteilhaft kann eine Bewegungseinrichtung zum Bewegen der zweiten Wandung über Antriebe, wie beispielsweise Spindelantriebe, verfügen, welche beispielsweise zu Wartungszwecken ein Absenken der zweiten Wandung gegenüber der ersten Wandung ermöglichen.

Bei einer weiteren bevorzugten Ausführungsform weist die Abschirmeinrichtung wenigstens abschnittsweise eine dritte Wandung auf, welche seitlich neben dem Transportpfad der Behältnisse verläuft, wobei der Transportpfad wenigstens abschnittsweise zwischen der ersten Wandung und der dritten Wandung verläuft. Bei dieser Ausführungsform bilden die erste Wandung und die dritte Wandung bevorzugt einen Kanal aus, innerhalb dessen die Kunststoffbehältnisse transportiert werden. Bevorzugt werden die Kunststoffbehältnisse während ihres Transports durch diesen Kanal an ihrer Außenoberfläche sterilisiert.

Bevorzugt ist dabei wenigstens eine Sterilisationseinrichtung in die erste und/oder die dritte Wandung integriert. Bei einer weiteren bevorzugten Ausführungsform ist wenigstens eine Sterilisationseinrichtung in die erste Wandung integriert und wenigstens eine Sterilisationseinrichtung in die dritte Wandung. Bevorzugt sind dabei die beiden Sterilisationseinrichtungen entlang des Transportpfades der Kunststoffvorformlinge versetzt zueinander angeordnet.

Bei einer weiteren vorteilhaften Ausführungsform ist die dritte Wandung gegenüber der ersten Wandung beweglich. Dies ist insbesondere vorteilhaft, um durch die besagte Bewegung der dritten Wandung gegenüber der ersten Wandung den Kanal zwischen diesen Wandungen zugänglich zu machen und insbesondere auch die Sterilisationseinrichtungen, welche in diesen Wandungen angeordnet sind.

Bevorzugt ist die dritte Wandung an der zweiten Wandung bzw. d.h. der Bodenwandung angeordnet und/oder eine Bewegung der dritten Wandung ist an eine Bewegung der zweiten Wandung gekoppelt. Falls also die zweite Wandung abgesenkt wird, wird damit gleichzeitig auch die dritte Wandung gegenüber der ersten Wandung abgesenkt und somit wird gleichzeitig auch der Kanal zwischen der ersten Wandung und der dritten Wandung geöffnet.

Bevorzugt ist die dritte Wandung einteilig mit dem Boden verbunden. Auf diese Weise kann der Kanal zwischen den Wandungen geöffnet werden. Bevorzugt ist die dritte Wandung daauch in der Längsrichtung der Behältnisse bewegbar. Bevorzugt verläuft der Kanal zwischen der ersten Wandung und der dritten Wandung wenigstens abschnittsweise entlang einer Kreislinie und bezogen auf diese Kreislinie ist besonders bevorzugt die dritte Wandung innerhalb der ersten Wandung angeordnet.

Die vorliegende Erfindung ist weiterhin auf ein Verfahren zum Behandeln von Kunststoffbehältnissen nach Anspruch 7 gerichtet.

Erfindungsgemäß weist die Abschirmeinrichtung eine zweite Wandung auf, welche unterhalb des Transportpfades der Kunststoffbehältnisse ausgebildet ist und es wird in einem weiteren Betriebszustand die zweite Wandung bewegt, um wenigstens einen Bereich der Sterilisationseinrichtung und insbesondere einen Innenbereich, in welchem auch der Transportpfad der Kunststoffbehältnisse verläuft, für einen Benutzer zugänglich zu machen.

Vorteilhaft wird daher ein Isolator ausgebildet, innerhalb dessen die Kunststoffbehältnisse transportiert werden, wobei dieser Isolator zumindest auch durch diese besagte zweite Wandung abgegrenzt ist. Vorteilhaft weist die Abschirmeinrichtung zwei Wandungen auf, welche den Transportpfad der Kunststoffbehältnisse seitlich begrenzen sowie die oben erwähnte zweite Wandung, welche die Kunststoffvorformlinge bzw. deren Transportpfad auch nach unten hin begrenzt.

Bei einer weiteren vorteilhaften Ausführungsform werden die Kunststoffbehältnisse zu ihrer Sterilisation durch einen Transportkanal gefördert, die durch wenigstens zwei Seitenwände und die zweite Wandung ausgebildet wird und durch die Bewegung der zweiten Wandung wird eine unterhalb des Transportpfades befindliche Öffnung freigegeben, durch welche ein Innenbereich der Sterilisationseinrichtung für den Benutzer zugänglich gemacht wird.

Bei diesem Innenbereich handelt es sich insbesondere um einen Bereich, in dem Röntgenstrahlung entsteht, welche jedoch gegenüber einem von diesem Innenbereich abzugrenzenden Außenbereich abzuschirmen ist.

Insbesondere handelt es sich bei dem besagten zweiten Betrieb um einen Wartungsbetrieb.

Bei einem bevorzugten Verfahren wird sowohl eine Außenwandung der Kunststoffbehältnisse als auch eine Innenwandung der Kunststoffbehältnisse mit den Ladungsträgern bzw. der Strahlung beaufschlagt. Vorteilhaft handelt es sich bei den Ladungsträgern um Elektronen. Es könnten jedoch auch andere Ladungsträger vorgesehen sein, wie beispielsweise Alphateilchen oder Protonen. Unter Ladungsträger werden damit Partikel verstanden, welche über eine elektrische Ladung verfügen. Vorteilhaft ist der Transportpfad, entlang dessen die Kunststoffbehältnisse transportiert werden, kreisförmig bzw. kreissegmentförmig. Besonders bevorzugt handelt es sich bei den Kunststoffbehältnissen um Kunststoffvorformlinge, welche im Rahmen eines Expansionsvorgangs zu Kunststoffbehältnissen, beispielsweise Kunststoffflaschen, expandiert werden können.

Weitere Vorteile und Ausführungsformen ergeben sich aus den beigefügten Zeichnungen. Darin zeigen:
- Fig. 1: eine Darstellung einer Vorrichtung zum Sterilisieren von Behältnissen;
- Fig. 2: eine Darstellung einer Vorrichtung in einem geöffnetem Zustand; und
- Fig. 3: eine Detaildarstellung der in Fig. 2 gezeigten Vorrichtung.

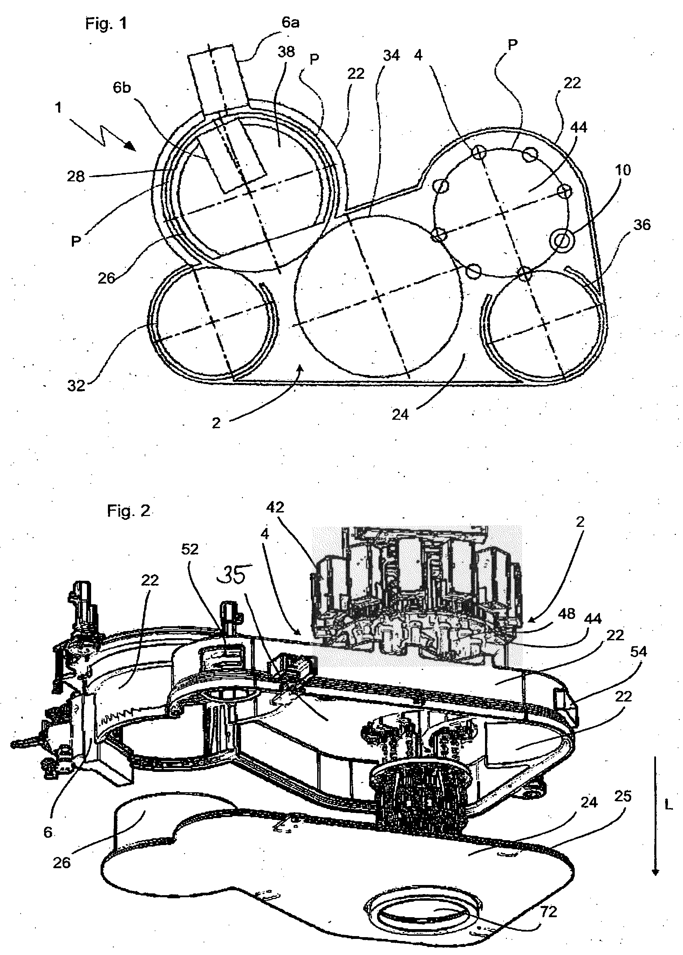

Fig. 1 zeigt eine Darstellung einer Vorrichtung 1 zum Sterilisieren von Kunststoffbehältnissen 10 und insbesondere Kunststoffvorformlingen. Dabei werden die Kunststoffvorformlinge 10 der Vorrichtung 1 über eine Zuführöffnung (nicht gezeigt) und eine Zuführeinrichtung 32, die hier als Zuführstern ausgeführt ist, zugeführt. Anschließend erfolgt eine Außensterilisation der Kunststoffvorformlinge. Diese werden dabei auf einem kreisförmigen Transportpfadabschnitt vorbei an zwei Außen-Steritisationseinrichtungen 6a und 6b geführt, welche eine Außenoberfläche der Kunststoffvorformlinge mit Ladungsträgern und insbesondere Elektronen bestrahlen. Das Bezugszeichen 38 kennzeichnet grob schematisch ein Transportelement wie einen Transportstern, der zum Transportieren der Kunststoffvorformlinge während deren Außensterilisation dient. Dieses Transportelement kann dabei einen drehbaren Träger aufweisen, an dem eine Vielzahl von Halteeinrichtungen zum Halten der Kunststoffbehältnisse angeordnet ist.

Anschließend werden die Kunststoffvorformlinge mit einem Transportelement 34 zu einer Innensterilisationseinrichtung transportiert. Bei diesem Transportelement 34 kann es sich um einen Transferstern handeln, der auch einen Teilungsverzug zwischen den einzelnen Kunststoffvorformlingen (10) ermöglicht. In dem Bereich der Außensterilisation wird der Transportpfad der Kunststoffvorformlinge sowohl von der Wandung 22 nach außen hin als auch von der Wandung 26 nach innen hin begrenzt.

Das Bezugszeichen 4 bezieht sich auf zweite Sterilisationseinrichtungen, die an einem gemeinsamen und drehbaren Träger 44 angeordnet sind. Diese zweiten Sterilisationseinrichtungen 4 weisen dabei jeweils stangenartige Körper auf, die in einen Innenraum der Kunststoffvorformlinge einführbar sind, um diese so mit den Ladungsträgern zu beaufschlagen. Diese stangenartigen Körper weisen dabei jeweils an ihrem unteren Ende ein Austrittsfenster auf, durch welches hindurch die Ladungsträger aus diesen stangenartigen Körpern austreten können. Das Bezugszeichen 2 bezieht sich auf die Transporteinrichtung in ihrer Gesamtheit, welche hier die oben beschriebenen einzelnen Transportelemente aufweist.

Sowohl bei der Außensterilisation als auch bei der Innensterilisation entsteht jedoch unerwünschte Röntgenstrahlung, die möglichst abgeschirmt werden sollte. Zu diesem Zweck weist die Vorrichtung eine Umfangswandung 22 auf, welche hier den vollständigen Transportpfad P der Kunststoffvorformlinge 10 umgibt. Diese Umfangswandung kann dabei beispielsweise aus Blei hergestellt sein. Nach oben hin wird die Strahlung durch einen Deckel 28 abgeschirmt und nach unten hin durch eine Bodenwandung 24. Das Bezugszeichen 36 kennzeichnet eine Abführeinrichtung, welche die sterilisierten Behältnisse 10 wieder aus der Vorrichtung 1 abführt. Auch diese Abführeinrichtung 36 kann dabei als Transportstern ausgebildet sein.

Fig. 2 zeigt eine weitere Darstellung einer Vorrichtung 1 zum Sterilisieren von Kunststoffvorformlingen 10. Man erkennt hier die erste Zuführöffnung 52, über welche die Kunststoffvorformlinge 10 der Vorrichtung 1 zugeführt werden und eine zweite Öffnung, über welche die sterilisierten Kunststoffvorformlinge abgeführt werden. Innerhalb des in seiner Gesamtheit mit 20 bezeichneten Gehäuses kann im Arbeitsbetrieb ein Reinraum ausgebildet sein, so dass die Sterilisation der Kunststoffvorformlinge ebenfalls unter Reinraumbedingungen erfolgt. Damit können sowohl die Zuführöffnung 52 als auch die Abführöffnung 54 (nicht gezeigte) Schleusenelemente aufweisen, welche auch zur Aufrechterhaltung eines derartigen Reinraums dienen.

Bei der in Fig. 2 gezeigten Darstellung ist die Bodenwandung 24 abgesenkt und damit ist der Innenraum der Vorrichtung für Wartungsarbeiten zugänglich. Man erkennt, dass der Wandungsabschnitt 26, der den Transportpfad P der Kunststoffvorformlinge nach innen hin begrenzt, nicht gemeinsam mit der Wandung 22 ausgebildet ist, sondern an der Bodenwandung 24 angeordnet ist. Auf diese Weise kann auch der relative enge Spalt, der sich im Arbeitsbetrieb zwischen den Wandungen 22 und 26 ergibt, geöffnet werden, in dem die beiden Wandungen 22 und 26 auseinandergeschoben werden. Die Bodenwandung 24 dient, wie oben ausgeführt, auch als Sitz- oder Standfläche für einen Benutzer, der so bequem an den einzelnen Elementen der Sterilisationsvorrichtung Wartungsarbeiten vornehmen kann. Das Bezugszeichen L bezieht sich auf die Bewegungsrichtung, in der das Bodenteil bzw. die Bodenwandung abgesenkt werden kann. Diese Richtung L ist dabei bevorzugt parallel zu den Längsrichtungen der transportierten Kunststoffbehältnisse. Daneben können zwischen der Bodenwandung 24 und der ersten Wandung noch Dichtungsmittel vorgesehen sein, welche in einem geschlossenen Zustand der Vorrichtung bzw. während eines Arbeitsbetriebs einen Austritt von Störstrahlung sicher verhindern. So könnte beispielsweise an dem Bodenteil 24 ein umlaufender und sich in Richtung der Seitenwand erstreckender Rand 25 vorgesehen sein, der in einem geschlossenen Zustand der Vorrichtung die Seitenwand 22 wenigstens teilweise und bevorzugt vollständig umgibt. Auch könnte ein derartiger Rand auch innerhalb der Seitenwand 22 angeordnet sein.

Die einzelnen Sterilisationseinrichtungen 4 weisen jeweils Elektronenerzeugungseinrichtungen 42 sowie auch Beschleunigungseinrichtungen auf, welche die Elektronen in Richtung der jeweiligen Austrittsfenster beschleunigen. Daneben sind die stangenartigen Körper 48 erkennbar, die in das Innere der Kunststoffvorformlinge einführbar sind. Das Bezugszeichen 35 kennzeichnet eine weitere stationäre Deckelwand, welche den Austritt von (Röntgen)Strahlung nach oben verhindert.

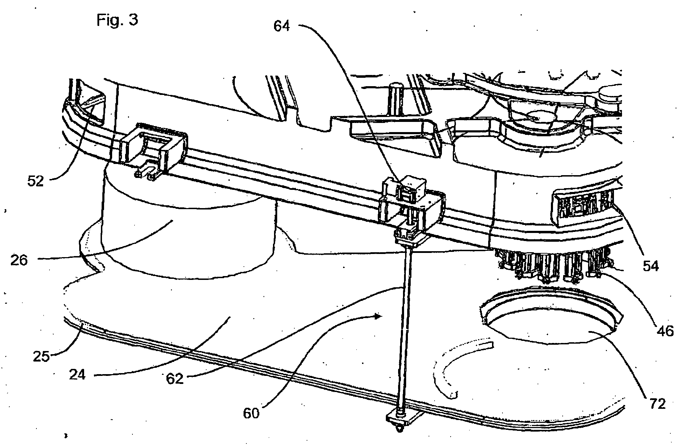

Fig. 3 zeigt eine vergrößerte Darstellung der in Fig. 2 gezeigten Vorrichtung. Hier ist eine Antriebseinrichtung 60 dargestellt, die zum Absenken und Anheben des Bodenteils 28 dient. Diese Antriebseinrichtung 60 weist eine Antriebsspindel 62 sowie ein Antriebselement 64 wie etwa einen Elektromotor auf. Insgesamt sind zum Senken und Heben des Bodenteils mehrere derartige Antriebseinrichtungen vorgesehen. Das Bezugszeichen 62 kennzeichnet eine in dem Bodenteil 24 angeordnete Öffnung und das Bezugszeichen 46 Halteeinrichtungen, die zum Halten der zu sterilisierenden Kunststoffvorformlinge dienen. Im Arbeitsbetrieb können die Kunststoffvorformlinge angehoben werden und so die Strahlfinger in diese eingeführt werden. Die Halteeinrichtungen 48 können beim Zustellen des Bodenteils 24 auf die Seitenwandung 22 durch die Öffnung 72 hindurchgeführt werden. Im Arbeitsbetrieb wäre es denkbar, dass die Behältnisse durch die Öffnung 72 in die Sterilisationskammer gehoben werden und durch diese Hubbewegung die Strahlfinger in die Kunststoffbehältnisse eingeführt werden. In einem ersten Betriebszustand - der Produktion bzw. Entkeimung - ist der Boden in der oberen Stellung, schließt also den Reinraum ab. In einem zweiten Betriebsstand - der Wartung - ist der Boden in dem heruntergefahrenen Zustand. Nachdem die Wartungsarbeiten abgeschlossen sind, wird der gesamte Reinraum wieder sterilisiert, bevor mit dem ersten Betriebszustand fortgefahren wird. Bevorzugt erfolgt diese Sterilisierung nach dem Hochfahren des Bodens bei der Bodenwartung.

### Bezugszeichenliste

- 1: Vorrichtung zum Sterilisieren von Kunststoffbehältnissen
- 10: Kunststoffbehältnis
- 22: seitliche Wandung
- 24: Bodenteil, Bodenwandung
- 25: Rand
- 26: seitliche Wandung
- 28: Deckel
- 32: Zuführeinrichtung
- 34: Transportelement
- 35: Deckelwand
- 36: Abführeinrichtung
- 38: Transportelement
- 42: Elektronenerzeugungseinrichtung
- 44: Träger
- 46: Halteeinrichtung
- 48: stangenartiger Körper
- 52: Zuführöffnung
- 54: Abführöffnung
- 60: Antriebseinrichtung
- 62: Antriebsspindel
- 64: Antriebselement
- 72: Öffnung

- P: Transportpfad

## Patentansprüche

1. Vorrichtung (1) zum Sterilisieren von Behältnissen (10) mit einer Transporteinrichtung (2), welche die zu sterilisierenden Behältnisse (10) entlang eines vorgegebenen Transport-pfads (P) transportiert, mit wenigstens einer Sterilsationseinrichtung (4, 6a, 6b), welche die Behältnisse (10) während ihres Transports zu ihrer Sterilisation mit Ladungsträgern beaufschlagt, wobei diese Sterilisationseinrichtung (4, 6a, 6b) eine Erzeugungseinrichtung (42) zum Erzeugen von Ladungsträgern aufweist, und mit einer Abschirmeinrichtung (20) zum Abschirmen von entstehender Strahlung, welche wenigstens abschnittsweise den Transportpfades (P) der Behältnisse umgibt, wobei
die Abschirmeinrichtung (20) eine erste Wandung (22) aufweist, welche seitlich neben dem Transportpfad der Behältnisse (10) angeordnet ist sowie eine zweite Wandung (24), welche unterhalb des Transportpfades der Behältnisse (10) angeordnet ist, **dadurch gekennzeichnet, dass** mittels einer Antriebseinrichtung die zweite Wandung (24) gegenüber dem Transportpfad (P) der Behältnisse (10) und auch gegenüber der ersten Wandung bewegbar ist, und wobei durch die Bewegung der zweiten Wandung (24) wenigstens eine Sterilisationseinrichtung (4, 6a, 6b) für Benutzereingriffe zugänglich machbar ist

2. Vorrichtung (1) nach Anspruch 1,
**dadurch gekennzeichnet, dass**
die zweite Wandung (24) wenigstens abschnittsweise als Stützfläche für einen Benutzer ausgebildet ist.

3. Vorrichtung (1) nach wenigstens einem der vorangegangenen Ansprüche,
**dadurch gekennzeichnet, dass**
die Transporteinrichtung (2) einen bewegbaren Träger (34, 44) aufweist, an dem eine Vielzahl von Halteelementen zum Halten der Behältnisse (10) angeordnet ist.

4. Vorrichtung (1) nach wenigstens einem der vorangegangenen Ansprüche,
**dadurch gekennzeichnet, dass**
die zweite Wandung (24) in einer Längsrichtung (L) der zu sterilisierenden Behältnisse (10) bewegbar ist.

5. Vorrichtung (1) nach wenigstens einem der vorangegangenen Ansprüche,
**dadurch gekennzeichnet, dass**
die Abschirmeinrichtung wenigstens abschnittsweise eine dritte Wandung (26) aufweist, welche seitlich neben dem Transportpfad (P) der Behältnisse (10) verläuft, wobei der Transportpfad (P) wenigstens abschnittsweise zwischen der ersten Wandung (22) und der dritten Wandung (26) verläuft.

6. Vorrichtung (1) nach Anspruch 5,
**dadurch gekennzeichnet, dass**
die dritte Wandung (26) gegenüber der ersten Wandung (22) beweglich ist.

7. Verfahren zum Behandeln von Kunststoffbehältnissen (10), wobei die Kunststoffbehältnisse (10) in einem Arbeitsbetrieb entlang eines vorgegebenen Transportpfades (P) transportiert und während dieses Transports zu ihrer Sterilisation von wenigstens einer Sterilisationseinrichtung (4) mit Ladungsträgern beaufschlagt werden, wobei diese Ladungsträger von einer Ladungsträgererzeugungseinrichtung (42) erzeugt und beschleunigt werden, wobei bei der Sterilisation der Kunststoffbehältnisse (10) entstehende Röntgenstrahlung von einer Abschirmeinrichtung (20) abgeschirmt werden und diese Abschirmeinrichtung (20) eine erste Wandung (22) aufweist, welche sich wenigstens abschnittsweise entlang des Transportpfades (P) der Kunststoffbehältnisse erstreckt,
**dadurch gekennzeichnet, dass**
die Abschirmeinrichtung (20) eine zweite Wandung (24) aufweist, welche unterhalb des Transportpfades der Kunststoffbehältnisse (10) ausgebildet ist und wobei in einem weiteren Betriebszustand die zweite Wandung (24) mittels einer Antriebseinrichtung gegenüber dem Transportpfad (P) der Behältnisse (10) und auch gegenüber der ersten Wandung bewegt wird, um wenigstens einen Bereich der Sterilisationseinrichtung für einen Benutzer zugänglich zu machen.

8. Verfahren nach Anspruch 7,
**dadurch gekennzeichnet, dass**
die Kunststoffbehältnisse (10) während ihrer Sterilisation durch einen Transportkanal gefördert werden, der durch wenigstens zwei Seitenwände (22, 26) und die zweite Wandung (24) ausgebildet wird und durch die Bewegung der zweiten Wandung eine unterhalb des Transportpfades befindliche Öffnung freigegeben wird, durch welche der Bereich der Sterilisationseinrichtung (1) für den Benutzer zugänglich gemacht wird.

9. Verfahren nach wenigstens einem der vorangegangenen Ansprüche,
**dadurch gekennzeichnet, dass**
sowohl eine Außenwandung als auch eine Innenwandung der Kunststoffbehältnisse mit den Ladungsträgern beaufschlagt wird.

## Claims

1. Apparatus (1) for sterilising containers (10), with a transport device (2) which transports the containers (10) to be sterilised along a predefined transport path (P), with at least one sterilisation device (4, 6a, 6b) which acts upon the containers (10) with charge carriers for their sterilisation during their transport, wherein this sterilisation device (4, 6a, 6b) has a generating device (42) for generating charge carriers, and with a screening device (20) for screening radiation occurring, which surrounds the transport path (P) of the containers at least in portions, wherein the screening device (20) has a first wall (22) which is arranged at the side next to the transport path of the containers (10) and a second wall (24) which is arranged below the transport path of the containers (10), wherein the second wall (24) is movable in relation to the transport path (P) of the containers (10) and also in relation to the first wall by means of a drive device and wherein the movement of the second wall (24) access to at least one sterilisation device (4, 6a, 6b) for operator intervention is made possible.

2. Apparatus (1) according to according to claim 1, **characterised in that** the second wall (24) is formed at least in portions as a support surface for an operator.

3. Apparatus (1) according to at least one of the preceding claims, **characterised in that** the transport device (2) has a movable carrier (34, 44) on which a plurality of holding elements is arranged for holding the containers (10).

4. Apparatus (1) according to at least one of the preceding claims, **characterised in that** the second wall (24) is movable in a longitudinal direction (L) of the containers (10) to be sterilised.

5. Apparatus (1) according to at least one of the preceding claims, **characterised in that** the screening device at least in portions has a third wall (26) which extends at the side next to the transport path (P) of the containers (10), wherein the transport path (P) at least in portions extends between the first wall (22) and the third wall (26).

6. Apparatus (1) according to claim 5, **characterised in that** the third wall (26) is movable in relation to the first wall (22).

7. Method for treating plastics material containers (10), wherein in a working mode, the plastics material containers (10) are transported along a predefined transport path and during this transport acted upon with charge carriers for their sterilisation by at least one sterilisation device (4), wherein these charge carriers are generated and accelerated by a charge carrier generating device (42), wherein X-ray radiation occurring on sterilisation of the plastics material containers (10) is screened by a screening device (20) and this screening device (20) has a first wall (22) which extends at least in portions along the transport path of the plastics material containers, **characterised in that** the screening device (20) has a second wall (24) which is formed below the transport path of the plastics material containers (10), and wherein in a further operating mode, the second wall (24) is moved by means of a drive device in relation to the transport path (P) of the containers (10) and also in relation to the first wall in order to create access to at least one region of the sterilisation device for an operator.

8. Method according to claim 7, **characterised in that** during their sterilisation, the plastics material containers (10) are transported through a transport channel which is formed by at least two side walls (22, 26) and the second wall (24), and the movement of the second wall exposes an opening located below the transport path, through which the region of the sterilisation device (1) is made accessible for the operator.

9. Method according to at least one of the preceding claims, **characterised in that** both an outer wall and an inner wall of the plastics material containers are acted upon with charge carriers.

## Revendications

1. Installation (1) pour la stérilisation de récipients (10), avec un dispositif de transport (2) convoyant les récipients (10) à stériliser le long d'un chemin de transport (P) défini, avec au moins un dispositif de stérilisation (4, 6a, 6b) soumettant les récipients (10) à des porteurs de charge pour leur stérilisation pendant leur transport, ledit dispositif de stérilisation (4, 6a, 6b) comprenant un dispositif générateur (42) pour la génération de porteurs de charge, et avec un dispositif de blindage (20) pour la protection contre le rayonnement formé, lequel entoure au moins en sections le chemin de transport (P) des récipients, où
le dispositif de blindage (20) comportant une première paroi (22) disposée latéralement au chemin de transport des récipients (10), et une deuxième paroi (24) disposée en dessous du chemin de transport des récipients (10), **caractérisée en ce que** la deuxième paroi (24) est déplaçable au moyen d'un dispositif d'entraînement, par rapport au chemin de transport (P) des récipients (10) et également par rapport à la première paroi, et **en ce que** le déplacement de la deuxième paroi (24) peut rendre accessible au moins un dispositif de stérilisation (4, 6a, 6b) pour des interventions d'utilisateur.

2. Installation (1) selon la revendication 1,
**caractérisée en ce que**
la deuxième paroi (24) est au moins en sections réalisée comme surface d'appui pour un utilisateur.

3. Installation (1) selon au moins une des revendications précédentes,
**caractérisée en ce que**
le dispositif de transport (2) comprend un support mobile (34, 44) sur lequel est disposée une pluralité d'éléments de maintien pour le maintien des récipients (10).

4. Installation (1) selon au moins une des revendications précédentes,
**caractérisée en ce que**
la deuxième paroi (24) est déplaçable dans le sens de la longueur (L) des récipients (10) à stériliser.

5. Installation (1) selon au moins une des revendications précédentes,
**caractérisée en ce que**
le dispositif de blindage comprend au moins en sections une troisième paroi (26) qui s'étend latéralement au chemin de transport (P) des récipients (10), ledit chemin de transport (P) s'étendant au moins en sections entre la première paroi (22) et la troisième paroi (26).

6. Installation (1) selon la revendication 5,
**caractérisée en ce que**
la troisième paroi (26) est déplaçable par rapport à la première paroi (22).

7. Procédé de traitement de récipients en matière plastique (10), lesdits récipients en matière plastique (10) étant dans un mode de travail transportés le long d'un chemin de transport (P) défini et soumis par au moins un dispositif de stérilisation (4) à des porteurs de charge pour leur stérilisation pendant ce transport, lesdits porteurs de charge étant générés et accélérés par un dispositif générateur (42) de porteurs de charge, un dispositif de blindage (20) protégeant du rayonnement X formé lors de la stérilisation des récipients en matière plastique (10) et ledit dispositif de blindage (20) comportant une première paroi (22) s'étendant au moins en sections le long du chemin de transport (P) des récipients en matière plastique,
**caractérisé en ce que**
le dispositif de blindage (20) comporte une deuxième paroi (24) formée en dessous du chemin de transport des récipients en matière plastique (10) et **en ce que** dans un autre mode de service, la deuxième paroi (24) est déplacée par rapport au chemin de transport (P) des récipients (10) et également par rapport à la première paroi au moyen d'un dispositif d'entraînement, pour rendre accessible au moins une partie du dispositif de stérilisation à un utilisateur.

8. Procédé selon la revendication 7,
**caractérisé en ce que**
les récipients en matière plastique (10) sont convoyés par un canal de transport pendant leur stérilisation, lequel est formé par au moins deux parois latérales (22, 26) et la deuxième paroi (24), et **en ce qu'**une ouverture située en dessous du chemin de transport est dégagée par le déplacement de la deuxième paroi, par laquelle la partie du dispositif de stérilisation (1) est rendue accessible à l'utilisateur.

9. Procédé selon au moins une des revendications précédentes,
**caractérisé en ce qu'**
une paroi extérieure et une paroi intérieure des récipients en matière plastique sont soumises aux porteurs de charge.
